# EUROPEAN PATENT SPECIFICATION

(11) **EP 1 556 430 B1**
(45) Date of publication and mention of the grant of the patent: **24.06.2009**
(21) Application number: 03779244.7
(22) Date of filing: 23.10.2003
(51) Int. Cl.: C08G 18/12, C08G 18/42, C09J 175/06, A61L 24/04

(54) **BIOABSORBABLE ADHESIVE COMPOUNDS**
BIOLOGISCH ABSORBIERBARE ADHÄSIVE VERBINDUNGEN
COMPOSES ADHESIFS BIOABSORBABLES

(30) Priority: 28.10.2002 US 421881 P
(43) Date of publication of application: 27.07.2005
(73) Proprietor: Tyco Healthcare Group LP, Norwalk, Connecticut 06856 (US)
(72) Inventor: ROBY, Mark, Killingworth, CT 06419 (US)
(74) Representative: HOFFMANN EITLE
(86) International application number: PCT/US2003/033826
(87) International publication number: WO 2004/039857

(56) References cited:
- EP-A- 0 077 192
- US-A- 3 773 595
- US-A- 4 323 491
- US-A- 4 804 691
- US-A- 4 804 691

## Description

### Technical Field

This disclosure relates to bioabsorbable compounds and compositions useful as surgical adhesives and sealants.

### Description of the Related Art

In recent years there has developed increased interest in replacing or augmenting sutures with adhesive bonds. The reasons for this increased interest include: (1) the potential speed with which repair might be accomplished; (2) the ability of a bonding substance to effect complete closure, thus preventing seepage of fluids; and (3) the possibility of forming a bond without excessive deformation of tissue.

Studies in this area, however, have revealed that, in order for surgical adhesives to be accepted by surgeons, they must possess a number of properties. First, they must exhibit high initial tack and an ability to bond rapidly to living tissue. Secondly, the strength of the bond should be sufficiently high to cause tissue failure before bond failure. Thirdly, the adhesive should form a bridge, preferably a permeable flexible bridge. Fourthly, the adhesive bridge and/or its metabolic products should not cause local histotoxic or carcinogenic effects.

A number of adhesive systems such as alkyl cyanoacrylates, polyacrylates, maleic anhydride/methyl vinyl ethers, epoxy systems, polyvinyl alcohols, formaldehyde and gluteraldehyde resins and isocyanates have been investigated as possible surgical adhesives. None has gained acceptance because each fails to meet one or more of the criteria noted above. The principal criticism of these systems has been the potential toxicity problems they pose.

EP-0077192 discloses adhesive compositions comprising trifunctional compounds capped with aromatic diisocyanates and an aromatic triisocyanate.

It would be desirable to provide novel metabolically-acceptable bioabsorbable diisocyanate-based adhesives and in particular metabolically-acceptable surgical adhesives. It would also be desirable to provide metabolically-acceptable surgical adhesives which are biodegradable. It would also be desirable to provide a method for closing wounds in living tissue by use of novel, metabolically-acceptable surgical adhesives which are low in toxicity as a consequence of their physical properties.

### Summary

The present compositions, upon curing, provide a bioabsorbable adhesive or sealant suitable for use in medical or surgical applications. These compositions contain three compounds. The first compound is an isocyanate-endcapped absorbable oligomer. To make their first component, an absorbable oligomeric material is prepared by polymerizing one or more hydrolyzable monomers in the presence of a bifunctional or multifunctional initiator. This oligomer is then reacted with an aromatic diisocyanate to terminate, or end-cap, the oligomer. The second compound is a trifunctional compound that is also diisocyanate terminated, or end-capped. The third compound is an aromatic diisocyanate. The three compounds are combined to form the present compositions.

The bioabsorbable compounds and compositions described herein are useful as surgical adhesives and/or sealants for joining portions of body tissue together or for joining surgically implantable devices to body tissue.

### DETAILED DESCRIPTION OF PREFERRED EMBODIMENT(S)

The compositions in accordance with the present disclosure include a) an isocyanate end-capped bioabsorbable oligomer; b) an isocyanate-endcapped trifunctional compound and c) an aromatic diisocyanate.

The first step in preparing the isocyanate end-capped bioabsorbable oligomer of the present composition is to polymerize hydrolyzable monomers in the presence of bi-or multi-functional initiators to prepare a compound, having the following structure:

[A]ₙ-X (II)

wherein A is a bioabsorbable group and is preferably derived from one or more monomers known to form a bioabsorbable polymer, n is from 1 to about 6 and X is a residue from the multifunctional initiator. Suitable monomers from which the bioabsorbable group can be derived include glycolic acid, glycolide, lactic acid, lactide, 1,4-dioxane-2-one, 1,3-dioxane-2-one, ε-caprolactone. Examples of suitable initiators include, but are not limited to, diols, such as, ethylene glycol, diethylene glycol, 1,3-propanediol, 1,4-butanediol, 1,5-pentanediol, 1,6-hexanediol, 1,7-heptanediol, 1,8-octanediol, 1,10-decanediol, 1,12-dodecanediol, 1,2-decanediol, 1,2-dodecanediol, 1,2-hexadecanediol, neopentyl glycol, 3-methyl-1,5-pentanediol, 2-methyl-1,3-propanediol, 2-butyl-2-ethyl-1,3-propanediol, 2-ethyl- 3-butyl- 1,3-propanediol, 2-ethyl-1,6-hexanediol; aromatic and alkyl triols, such as, for example, glycerol and 1,1,1-trimethylolpropane; polyols, such as neopentyl glycol, and pentaerythritol; alcohol amines, such as triethanolamine, 1-, and 2-aminopropanols, 2- and 4-aminobutanols, dicarboxylic acids such as succinic acid, glutaric acid, adipic acid, suberic acid, sebacic acid, dodecanedioic acid, and 2-ethyl-2-methylsuccinic acid; aromatic dicarboxylic acids, such as phthalic acid, isophthalic acid, and terephthalic acid.

Conditions for polymerizing hydrolyzable monomers in the presence of multifunctional initiators are within the purview or those skilled in the art. For example, the bioabsorbable oligomer can be prepared by drying purified monomer(s) used to form the bioabsorbable oligomer and then polymerizing at temperatures ranging from about 20°C. to about 220° C., preferably above 75° C., in the presence of an organometallic catalyst such as stannous octoate, stannous chloride, diethyl zinc or zirconium acetylacetonate. The polymerization time may range from 1 to 100 hours or longer depending on the other polymerization parameters but generally polymerization times of about 12 to about 48 hours are employed. In addition, a multifunctional initiator is employed. Generally, the amount of initiator used will range from about 0.01 to about 30 percent by weight based on the weight of the monomer. Preferably, the initiator will be present in the reaction mixture in an amount from about 0.5 to about 20 weight percent based on the weight of the monomer.

Once the oligomer is prepared, it is end-capped by reacting with an aromatic diisocyanate. Suitable aromatic diisocyanates include, but are not limited to, 1,4-diisocyanatobenzene, 1,1'-methylenebis[4-isocyanatobenzene], 2,4-diisocyanato-1-methylbenzene, 1,3-diisocyanato-2-methylbenzene, 1,5-diisocyanatonaphthalene, 1,1'-(1-methylethylidene)bis[4-isocyanatobenzene) and 1,3- and 1,4-bis(1-isocyanato-1-methylethyl)benzene.

Conditions for reacting hydroxyl-terminated oligomers with aromatic diisocyanates are within the purview of those skilled in the art. The conditions under which the oligomer is reacted with the diisocyanate may vary widely depending on the specific oligomer being endcapped, the specific diisocyanate being employed, and the desired degree of end capping to be achieved. Normally, the polymer is dissolved in a solvent and added dropwise to a solution of the diisocyanate at room temperature with stirring. The amount of diisocyanate employed can range from about 2 to about 8 moles of diisocyanate per mole of oligomer. Suitable reaction times and temperatures range from about 15 minutes to 72 hours or more at temperatures ranging from about 0°C. to 250° C.

Those skilled in the art will readily envision other reaction schemes for preparing useful isocyanate end-capped bioabsorbable oligomers.

The second component of the present compositions is a trifunctional compound that has end-capped with a disocyanate. Suitable trifunctional compounds include but are not limited to aromatic and alkyl triols, such as, for example, glycerol, and trimethylol propane; and alcohol amines, such as triethanolamine, 1-, and 2-aminopropanols, 2- and 4-aminobutanols. The trifunctional compound is preferably glycerol. The trifunctional compound is reacted with an aromatic diisocyante. Suitable examples of aromatic diisocyanates include, but are not limited to, aromatic polyisocyanates containing 6 to 20 carbon atoms, not including the carbon atoms in the NCO groups, such as o-, m- and p-phenylene diisocyanates (hereinafter referred to as PDI), 2,4- and 2,6-tolylene diisocyanates (TDI), diphenylmethane-2,4'-and 4,4'-diisocyanates (MDI), naphthalene-1,5-diisocyanate, triphenylmethane-4,4',4"-Misocyanate, polymethylene polyphenylenepoly-isocyanates (PAPI) obtained by phosgenation of anilineformidehyde condensation products, m- and p isocyanatophenyl sulfonyl isocyanate araliphatic polyisocyanates containing 8 to 15 carbon atoms, such as xylylene diisocyanates, diethylbenzene diisocyanates; and modified polyisocyanates of these polyisocyanates, containing urethane, carbodiimide, allophanate, urea, biuret, urethdione, urethimine, isocyanurate and/or oxazolidone groups, such as urethane-modified TDI, carbodiimide-modified MDI, urethane-modified MDI; as well as mixtures of two or more of them. Among these polyisocyanates, preferred are aromatic polyisocyanates (preferably diisocyanates), particularly PDI, TDI (including the 2,4- and 2,6-isomers, mixtures of them and crude TDI), MDI (including the 4,4'- and 2,4'-isomers, mixtures of them and crude MDI or PAPI), and modified polyisocyanates containing urethane, carbodiimide, allophanate, urea, biuret and/or isocyanurate groups, derived from PDI, TDI and/or MDI.

Reaction conditions suitable for end-capping the trifunctional compound with the diisocyanate are within the knowledge of those skilled in the art. The specific conditions employed will vary depending on a number of factors including the particular trifunctional compound chosen and the particular diisocyanate employed. Typically, a solution of the trifunctional compound is added dropwise to a solution of the diisocyanate at room temperature with stirring. The amount of diisocyanate employed can range from about 2 to about 8 moles of diisocyanate per mole of trifunctional compound. Suitable reaction times and temperatures range from about 15 minutes to 72 hours or more at temperatures ranging from about 0° C. to 250° C.

The third component of the present compositions is an aromatic diisocyanate compound. A non-exhaustive list of suitable diisocyanate compounds is provided above with respect to the preparation of the first two components.

The relative proportions of the three components in weight percent based on the total weight of the composition is set forth in the following table.

| Component | General Range | Preferred Range |
|---|---|---|
| End-capped Oligomer | 50 to 95% | 70 to 90% |
| End-capped | 5 to 40% | 8 to 25% |
| Trifunctional Compound | | |
| Aromatic Diisocyanate | 1 to 10% | 2 to 5% |

The present compositions can be prepared by simply mixing the three components together with stirring. Care should be taken not to contact the composition with water to avoid pre-mature crosslinking and the resulting thickening of the composition.

Upon crosslinking, the present bioabsorbable compounds can be used as tissue adhesives or sealants. Cross-linking is normally performed by exposing the composition to water, optionally in the presence of a catalyst.

The exact reaction conditions for achieving cross-linking will vary depending on a number of factors such as the particular bioabsorbable oligomer employed, the particular trifunctional compound employed, the particular aromatic diisocyanate employed and the relative amounts of the three components in the composition. Normally, the cross-linking reaction is conducted at temperatures ranging from 20° C. to about 40° C. for thirty seconds to about one hour or more. The amount of water employed will normally range from about 0.05 moles to 1 moles per mole of bioabsorbable compound. While water is a preferred reactant to effect cross-linking it should be understood that other compounds could also be employed either together with or instead of water. Such compounds include diethylene glycol and polyethylene glycol. When present, suitable catalysts for use in the cross-linking reaction include 1,4 diazobicyclo [2.2.2]octane, triethylamine, and diethylaminoethanol. The amount of catalyst employed can range from about 0.005 grams to about 5.0 grams per kilogram of compound being cross-linked.

When the present composition is intended for implantation it is possible to effectuate cross-linking in situ using the water naturally present in a mammalian body or with added water. However, to more precisely control the conditions and extent of cross-linking, it may be advantageous to partially cross-link the compound prior to its use as an implant.

The bioabsorbable compounds and compositions described herein are advantageously useful as a surgical adhesive or sealant, for example, for joining portions of body tissue together, or for adhering a surgical device such as a surgical mesh, fastener, implant, etc., to soft body tissue.

## Claims

1. A composition comprising:
a bioabsorbable oligomeric compound that is end-capped with an aromatic diisocyanate;
a trifunctional compound that is end-capped with an aromatic diisocyanate; and
an aromatic diisocyanate.

2. A composition as in claim 1 wherein the bioabsorbable oligomeric compound is a compound having the structure:
[A]ₙ-X
wherein A is a bioabsorbable group, n is from 1 to 6 and X is a residue from a multifunctional initiator.

3. A composition as in claim 2 wherein the bioabsorbable group is a group derived from a monomer selected from the group consisting of glycolic acid, glycolide, lactic acid, lactide, 1,4-dioxane-2-one, 1,3-dioxane-2-one and ε-caprolactone.

4. A composition as in claim 2 wherein X is a residue from a multifunctional initiator selected from the group consisting of diols, aromatic and alkyl triols, polyols, alcohol amines, dicarboxylic acids and aromatic dicarboxylic acids.

5. A composition as in claim 2 wherein X is a residue from a multifunctional initiator selected from the group consisting of ethylene glycol, diethylene glycol, 1,3-propanediol, 1,4-butanediol, 1,5-pentanediol, 1,6-hexanediol, 1,7-heptanediol, 1,8-octanediol, 1,10-decanediol, 1,12-dodecanediol, 1,2-decanediol, 1,2-dodecanediol, 1,2-hexadecanediol, neopentyl glycol, 3-methyl-1,5-pentanediol, 2-methyl-1,3-propanediol, 2-butyl-2-ethyl-1,3-propanediol, 2-ethyl-3-butyl- 1,3-propanediol, 2-ethyl-1,6-hexanediol, glycerol, 1,1,1-trimethylolpropane, neopentyl glycol, pentaerythritol, triethanoiamine, 1-aminopropanols, 2-aminopropanols, 2- aminobutanols, 4-aminobutanols, succinic acid, glutaric acid, adipic acid, suberic acid, sebacic acid, dodecanedioic acid, 2-ethyl-2-methylsuccinic acid, phthalic acid, isophthalic acid, and terephthalic acid.

6. A composition as ins claim 1 wherein the bioabsorbable oligomer is endcapped with an aromatic diisocyanate selected from the group consisting of 1,4-diisocyanatobenzene, 1,1'-methylenebis[4-isocyanatobenzene], 2,4-diisocyanato-1-methylbenzene, 1,3-diisocyanato-2-methylbenzene, 1,5-diisocyanatonaphthalene, 1,1'-(1-methylethylidene)bis[4-isocyanatobenzene) and 1,3- and 1,4-bis(1-isocyanato-1-methylethyl)benzene.

7. A composition as in claim 1 wherein the trifunctional compound is selected from the group consisting of glycerol, 1,1,1-trimethylolpropane, triethanolamine, 1-aminopropanols, 2-aminopropanols, 2-aminobutanols, 4-aminobutanols.

8. A composition as in claim 1 wherein the trifunctional compound is end-capped with an aromatic diisocyanate selected from the group consisting of:
(a) aromatic polyisocyanates containing 6 to 20 carbon atoms, not including the carbon atoms in the NCO groups;
(b) araliphatic polyisocyanates containing 8 to 15 carbon atoms; and
(c) modified polyisocyanates of these polyisocyanates, containing urethane, carbodiimide, allophanate, urea, biuret, urethdione, urethimine, isocyanurate and/or oxazolidone groups.

9. A composition as in claim 1 wherein the aromatic diisocyanate is selected from the group consisting of 1,4-diisocyanatobenzene, 1,1'-methylenebis[4-isocyanatobenzene], 2,4-diisocyanato-1-methylbenzene, 1,3-diisocyanato-2-methylbenzene, 1,5-diisocyanatonaphthalene, 1,1'-(1-methylethylidene)bis[4-isocyanatobenzene) and 1,3- and 1,4-bis(1-isocyanato-1-methylethyl)benzene.

10. A composition as in claim 1 wherein the bioabsorbable oligomeric compound that is end-capped with an aromatic diisocyanate is present in an amount from 50 to 95 percent by weight of the composition;
the trifunctional compound that is end-capped with an aromatic diisocyanate is present in an amount from 5 to 40 percent by weight of the composition; and
the aromatic diisocyanate is present in an amount from 1 to 10 percent by weight of the composition.

11. A composition as in claim 1 wherein the bioabsorbable oligomeric compound that is end-capped with an aromatic diisocyanate is present in an amount from 70 to 90 percent by weight of the composition;
the trifunctional compound that is end-capped with an aromatic diisocyanate is present in an amount from 8 to 25 percent by weight of the composition; and
the aromatic diisocyanate is present in an amount from 2 to 5 percent by weight of the composition.

12. A composition as in claim 1, which is for adhering first and second tissue surfaces, in a method comprising approximating the first and second tissue surfaces; and applying to the approximated tissue surfaces a composition of claim 1.

13. A composition as in claim 1, which is for adhering a surgical device to tissue, in a method comprising: applying to the surgical device a composition of claim 1; and contacting the surgical device with tissue.

14. A composition as in claim 1, which is for sealing a defect in tissue in a method comprising identifying a tissue site containing a defect; and applying a composition of claim 1 to the site of the defect.

15. A composition as in claim 1, which is for reducing leakage of bodily fluids or air in a method comprising applying to a tissue defect a composition in accordance with claim 1 and crosslinking the composition.

16. A composition as in claim 1 wherein:
(i) the bioabsorbable oligomeric compound has the structure:
[A]ₙ-X
wherein A is a bioabsorbable group derived from a monomer selected from the group consisting of glycolic acid, glycolide, lactic acid, lactide, 1,4-dioxane-2-one, 1,3-dioxane-2-one and ε-caprolactone, n is from about 1 to about 6 and X is a residue from a multifunctional initiator selected from the group consisting of ethylene glycol, diethylene glycol, 1,3-propanediol, 1,4-butanediol, 1,5-pentanediol, 1,6-hexanediol, 1,7-heptanediol, 1,8-octanediol, 1,10-decanediol, 1,12-dodecanediol, 1,2-decanediol, 1,2-dodecanediol, 1,2-hexadecanediol, neopentyl glycol, 3-methyl-1,5-pentanediol, 2-methyl-1,3-propanediol, 2-butyl-2-ethyl-1,3-propanediol, 2-ethyl- 3-butyl- 1,3-propanediol, 2-ethyl-1,6-hexanediol, glycerol, 1,1,1-trimethylolpropane, neopentyl glycol, pentaerythritol, triethanolamine, 1- aminopropanols, 2-aminopropanols, 2- aminobutanols, 4-aminobutanols, succinic acid, glutaric acid, adipic acid, suberic acid, sebacic acid, dodecanedioic acid, 2-ethyl-2-methylsudcinic acid, phthalic acid, isophthalic acid, and terephthalic acid;
(ii) the trifunctional compound is selected from the group consisting of glycerol 1,1,1-trimethylolpropane, triethanolamine 1-aminopropanols, 2-aminop'ropanols, 2- aminobutanols, 4-aminobutanols; and
(iii) the aromatic diisocyanate is selected from the group consisting of:
(a) aromatic polyisocyanates containing 6 to 20 carbon atoms, not including the carbon atoms in the NCO groups;
(b) araliphatic polyisocyanates containing 8 to 15 carbon atoms; and
(c) modified polyisocyanates of these polyisocyanates, containing urethane, carbodiimide, allophanate, urea, biuret, urethdione, urethimine, isocyanurate and/or oxazolidone groups.

17. A composition as in any of claims 12 -14, wherein the method comprises
(i) applying to tissue a composition as defined in claim 1; and
(ii) crosslinking the composition.

18. A composition as in claim 15 or claim 17, wherein crosslinking comprises contacting the composition with a compound selected from the group consisting of water, diethylene glycol and polyethylene glycol.

19. A composition as in claim 15 or claim 17, wherein crosslinking includes the use of a catalyst.

20. A composition as in claim 15 or claim 17 wherein crosslinking is conducted at temperatures from 20°C. to 40° C. for a time from thirty seconds to one hour.

21. A composition as in claim 15 or claim 17, wherein crosslinking is conducted at least partially prior to application to tissue.

## Patentansprüche

1. Zusammensetzung, die umfasst:
eine bioabsorbierbare oligomere Verbindung, die mit einem aromatischen Diisocyanat endverschlossen ist;
eine trifunktionelle Verbindung, die mit einem aromatischen Diisocyanat endverschlossen ist; und
ein aromatisches Diisocyanat.

2. Zusammensetzung gemäss Anspruch 1, in der die bioabsorbierbare oligomere Verbindung eine Verbindung mit der Struktur:
[A]ₙ-X
ist, worin A eine bioabsorbierbare Gruppe ist, n 1 bis 6 ist und X ein Rest aus einem multifunktionellen Starter ist.

3. Zusammensetzung gemäss Anspruch 2, in der die bioabsorbierbare Gruppe eine Gruppe ist, die abgeleitet ist von einem Monomer, ausgewählt aus der Gruppe bestehend aus Glykolsäure, Glycolid, Milchsäure, Lactid, 1,4-Dioxan-2-on, 1,3-Dioxan-2-on und ε-Caprolacton.

4. Zusammensetzung gemäss Anspruch 2, in der X ein Rest aus einem multifunktionellen Starter ist, ausgewählt aus der Gruppe bestehend aus Diolen, aromatischen und Alkyltriolen, Polyolen, Alkoholaminen, Dicarbonsäuren und aromatischen Dicarbonsäuren.

5. Zusammensetzung gemäss Anspruch 2, in der X ein Rest aus einem multifunktionellen Starter ist, ausgewählt aus der Gruppe bestehend aus Ethylenglykol, Diethylenglykol, 1,3-Propandiol, 1,4-Butandiol, 1,5-Pentandiol, 1,6-Hexandiol, 1,7-Heptandiol, 1,8-Octandiol, 1,10-Decandiol, 1,12-Dodecandiol, 1,2-Decandiol, 1,2-Dodecandiol, 1,2-Hexadecandiol, Neopentylglykol, 3-Methyl-1,5-pentandiol, 2-Methyl-1,3-propandiol, 2-Butyl-2-ethyl-1,3-propandiol, 2-Ethyl-3-butyl-1,3-propandiol, 2-Ethyl-1,6-hexandiol, Glycerin, 1,1,1-Trimethylolpropan, Neopentylglykol, Pentaerythrit, Triethanolamin, 1-Aminopropanolen, 2-Aminopropanolen, 2-Aminobutanolen, 4-Aminobutanolen, Bernsteinsäure, Glutarsäure, Adipinsäure, Suberinsäure, Sebacinsäure, Dodecandisäure, 2-Ethyl-2-methylbernsteinsäure, Phthalsäure, Isophthalsäure und Terephthalsäure.

6. Zusammensetzung gemäss Anspruch 1, in der das bioabsorbierbare Oligomer mit einem aromatischen Diisocyanat endverschlossen ist, das ausgewählt ist aus der Gruppe bestehend aus 1,4-Diisocyanatobenzol, 1,1'-Methylenbis[4-isocyanatobenzol], 2,4-Diisocyanato-1-methylbenzol, 1,3-Diisocyanato-2-methylbenzol, 1,5-Diisocyanatonaphthalin, 1,1'-(1-Methylethyliden)bis[4-isocyanatobenzol] und 1,3- und 1,4-Bis (1-isocyanato-1-methylethyl) benzol.

7. Zusammensetzung gemäss Anspruch 1, in der die trifunktionelle Verbindung ausgewählt ist aus der Gruppe, bestehend aus Glycerin, 1,1,1-Trimethylolpropan, Triethanolamin, 1-Aminopropanolen, 2-Aminopropanolen, 2-Aminobutanolen und 4-Aminobutanolen.

8. Zusammensetzung gemäss Anspruch 1, in der die trifunktionelle Verbindung mit einem aromatischen Diisocyanat endverschlossen ist, das ausgewählt ist aus der Gruppe, bestehend aus:
(a) aromatischen Polyisocyanaten, die 6 bis 20 Kohlenstoffatome enthalten, nicht eingeschlossen die Kohlenstoffatome in den NCO-Gruppen;
(b) araliphatischen Polyisocyanaten, die 8 bis 15 Kohlenstoffatome enthalten; und
(c) modifizierten Polyisocyanaten dieser Polyisocyanate, die Urethan-, Carbodiimid-, Allophanat-, Harnstoff-, Biuret-, Urethdion-, Urethimin-, Isocyanurat- und/oder Oxazolidongruppen enthalten.

9. Zusammensetzung gemäss Anspruch 1, in der das aromatische Diisocyanat ausgewählt ist aus der Gruppe, bestehend aus 1,4-Diisocyanatobenzol, 1,1'-Methylenbis[4-isocyanatobenzol], 2,4-Diisocyanato-1-methylbenzol, 1,3-Diisocyanato-2-methylbenzol, 1,5-Diisocyanatonaphthalin, 1,1'-(1-Methylethyliden)bis[4-isocyanatobenzol] und 1,3- und 1,4-Bis(1-isocyanato-1-methylethyl)benzol.

10. Zusammensetzung gemäss Anspruch 1, in der die bioabsorbierbare oligomere Verbindung, die mit einem aromatischen Diisocyanat endverschlossen ist, in einer Menge von 50 bis 95 Gew.% der Zusammensetzung vorliegt;
die trifunktionelle Verbindung, die mit einem aromatischen Diisocyanat endverschlossen ist, in einer Menge von 5 bis 40 Gew.% der Zusammensetzung vorliegt; und
das aromatische Diisocyanat in einer Menge von 1 bis 10 Gew.% der Zusammensetzung vorliegt.

11. Zusammensetzung gemäss Anspruch 1, in der die bioabsorbierbare oligomere Verbindung, die mit einem aromatischen Diisocyanat endverschlossen ist, in einer Menge von 70 bis 90 Gew.% der Zusammensetzung vorliegt;
die trifunktionelle Verbindung, die mit einem aromatischen Diisocyanat endverschlossen ist, in einer Menge von 8 bis 25 Gew.% der Zusammensetzung vorliegt; und
das aromatische Diisocyanat in einer Menge von 2 bis 5 Gew.% der Zusammensetzung vorliegt.

12. Zusammensetzung gemäss Anspruch 1, die zum Aneinanderkleben von ersten und zweiten Gewebeoberflächen in einem Verfahren dient, das das Annähern der ersten und zweiten Gewebeoberflächen und die Applikation einer Zusammensetzung von Anspruch 1 auf die angenäherten Gewebeoberflächen einschliesst.

13. Zusammensetzung gemäss Anspruch 1, die zum Ankleben einer chirurgischen Vorrichtung an Gewebe in einem Verfahren dient, das das Applizieren einer Zusammensetzung von Anspruch 1 auf die chirurgische Vorrichtung und das In-Kontakt-Bringen der chirurgischen Vorrichtung mit Gewebe umfasst.

14. Zusammensetzung gemäss Anspruch 1, die zum Verschliessen eines Defekts in einem Gewebe in einem Verfahren dient, das die Identifizierung einer Stelle im Gewebe, die einen Defekt enthält, und das Applizieren einer Zusammensetzung von Anspruch 1 auf die Stelle des Defekts umfasst.

15. Zusammensetzung gemäss Anspruch 1, die zur Verringerung der Leckage von Körperflüssigkeiten oder Luft in einem Verfahren dient, das die Applikation einer Zusammensetzung gemäss Anspruch 1 auf einen Gewebedefekt und das Vernetzen der Zusammensetzung umfasst.

16. Zusammensetzung gemäss Anspruch 1, in der:
(i) die bioabsorbierbare oligomere Verbindung die Struktur
[A]ₙ-X
hat, in der A eine bioabsorbierbare Gruppe ist, die abgeleitet ist von einem Monomer, ausgewählt aus der Gruppe bestehend aus Glykolsäure, Glycolid, Milchsäure, Lactid, 1,4-Dioxan-2-on, 1,3-Dioxan-2-on und ε-Caprolacton, n etwa 1 bis etwa 6 ist und X ein Rest aus einem multifunktionellen Starter ist, ausgewählt aus der Gruppe bestehend aus Ethylenglykol, Diethylenglykol, 1,3-Propandiol, 1,4-Butandiol, 1,5-Pentandiol, 1,6-Hexandiol, 1,7-Heptandiol, 1,8-Octandiol, 1,10-Decandiol, 1,12-Dodecandiol, 1,2-Decandiol, 1,2-Dodecandiol, 1,2-Hexadecandiol, Neopentylglykol, 3-Methyl-1,5-pentandiol, 2-Methyl-1,3-propandiol, 2-Butyl-2-ethyl-1,3-propandiol, 2-Ethyl-3-butyl-1,3-propandiol, 2-Ethyl-1,6-hexandiol, Glycerin, 1,1,1-Trimethylolpropan, Neopentylglykol, Pentaerythrit, Triethanolamin, 1-Aminopropanolen, 2-Aminopropanolen, 2-Aminobutanolen, 4-Aminobutanolen, Bernsteinsäure, Glutarsäure, Adipinsäure, Suberinsäure, Sebacinsäure, Dodecandisäure, 2-Ethyl-2-methylbernsteinsäure, Phthalsäure, Isophthalsäure und Terephthalsäure;
(ii) die trifunktionelle Verbindung ausgewählt ist aus der Gruppe bestehend aus Glycerin, 1,1,1-Trimethylolpropan, Triethanolamin, 1-Aminopropanolen, 2-Aminopropanolen, 2-Aminobutanolen, 4-Aminobutanolen; und
(iii) das aromatische Diisocyanat ausgewählt ist aus der Gruppe, bestehend aus:
(a) aromatischen Polyisocyanaten, die 6 bis 20 Kohlenstoffatome enthalten, nicht eingeschlossen die Kohlenstoffatome in den NCO-Gruppen;
(b) araliphatischen Polyisocyanaten, die 8 bis 15 Kohlenstoffatome enthalten; und
(c) modifizierten Polyisocyanaten dieser Polyisocyanate, die Urethan-, Carbodiimid-, Allophanat-, Harnstoff-, Biuret-, Urethdion-, Urethimin-, Isocyanurat- und/oder Oxazolidongruppen enthalten.

17. Zusammensetzung gemäss mindestens einem der Ansprüche 12 bis 14, wobei das Verfahren umfasst:
(i) Applikation einer Zusammensetzung, wie in Anspruch 1 definiert, auf Gewebe; und
(ii) Vernetzen der Zusammensetzung.

18. Zusammensetzung gemäss Anspruch 15 oder Anspruch 17, wobei die Vernetzung das In-Kontakt-Bringen der Zusammensetzung mit einer Verbindung, die ausgewählt ist aus der Gruppe, bestehend aus Wasser, Diethylenglykol und Polyethylenglykol, umfasst.

19. Zusammensetzung gemäss Anspruch 15 oder Anspruch 17, wobei das Vernetzen die Verwendung eines Katalysators einschliesst.

20. Zusammensetzung gemäss Anspruch 15 oder Anspruch 17, wobei das Vernetzen bei Temperaturen von 20 bis 40°C für eine Zeit von 30 Sekunden bis 1 Stunde durchgeführt wird.

21. Zusammensetzung gemäss Anspruch 15 oder Anspruch 17, wobei das Vernetzen zumindest teilweise vor der Applikation auf Gewebe durchgeführt wird.

## Revendications

1. Composition comprenant:
un composé oligomérique bioabsorbable qui est coiffé en extrémité avec un diisocyanate aromatique;
un composé trifonctionnel qui est coiffé en extrémité avec un diisocyanate aromatique; et
un diisocyanate aromatique.

2. Composition selon la revendication 1, dans laquelle le composé oligomérique bioabsorbable est un composé ayant la structure:
[A]ₙ-X
où A est un groupe bioabsorbable, n est de 1 à 6 et X est un résidu d'un initiateur multifonctionnel.

3. Composition selon la revendication 2, dans laquelle le groupe bioabsorbable est un groupe dérivé d'un monomère sélectionné dans le groupe consistant en acide glycolique, glycolide, acide lactique, lactide, 1,4-dioxane-2-one, 1,3-dioxane-2-one et ε-caprolactone.

4. Composition selon la revendication 2, dans laquelle X est un résidu d'un initiateur multifonctionnel sélectionné dans le groupe consistant en diols, triols aromatiques et alkyles, polyols, alcools amines, acides dicarboxyliques et acides dicarboxyliques aromatiques.

5. Composition selon la revendication 2, dans laquelle X est un résidu d'un initiateur multi-fonctionnel sélectionné dans le groupe consistant en éthylène glycol, diéthylène glycol, 1,3-propanediol, 1,4-butanediol, 1,5-pentanediol, 1,6-hexanediol, 1,7-hetpanediol, 1,8-octanediol, 1,10-décanediol, 1,12-dodécanediol, 1,2-décanediol, 1,2-dodécanediol, 1,2-hexadécanediol, néopentyl glycol, 3-méthyl-1,5-pentanediol, 2-méthyl-1,3-propanediol, 2-butyl-2-éthyl-1,3-propanediol, 2-éthyl-3-butyl-1,3-propanediol, 2-éthyl-1,6-hexanediol, glycérol, 1,1,1-triméthylpropane, néopentyl glycol, pentaérythritol, triéthanolamine, 1-aminopropanols, 2-aminopropanols, 2-aminobutanols, 4-aminobutanols, acide succinique, acide glutanique, acide adipique, acide subérique, acide sébacique, acide dodécanedioïque, acide 2-éthyl-2-méthylsuccinique, acide phtalique, acide isophtalique et acide téréphtalique.

6. Composition selon la revendication 1, dans laquelle l'oligomère bioabsorbable est coiffé en extrémité avec un diisocyanate aromatique sélectionné dans le groupe consistant en 1,4-diisocyanatobenzène, 1,1'-méthylènebis[4-isocyanatobenzène], 2,4-diisocyanato-1-méthylbenzène, 1,3-diisocyanato-2-méthylbenzène, 1,5-diisocyanatonaphtalène, 1,1'-(1-méthyléthylidène)bis[4-isocyanatobenzène) et 1,3- et 1,4-bis(1-isocyanato-1-méthyléthyl)benzène.

7. Composition selon la revendication 1, dans laquelle le composé trifonctionnel est sélectionné dans le groupe consistant en glycérol, 1,1,1-triméthylolpropane, triéthanolamine, 1-aminopropanols, 2-aminopropanols, 2-aminobutanols, 4-aminobutanols.

8. Composition selon la revendication 1, dans laquelle le composé trifonctionnel est coiffé en extrémité avec un diisocyanate aromatique sélectionné dans le groupe consistant en
(a) polyisocyanates aromatiques contenant 6 à 20 atomes de carbone, non incluant les atomes de carbone dans les groupes NCO;
(b) polyisocyanates araliphatiques contenant 8 à 15 atomes de carbone; et
(c) polyisocyanates modifiés de ces polyisocyanates, contenant des groupes d'uréthane, carbodiimide, allophanate, urée, biuret, urethdione, urethimine, isocyanurate et/ou oxazolidone.

9. Composition selon la revendication 1, dans laquelle le diisocyanate aromatique est sélectionné dans le groupe consistant en 1,4-diisocyanatobenzène, 1,1'-méthylènebis[4-isocyanatobenzène), 2,4-diisocyanato-1-méthylbenzène, 1,3-diisocyanato-2-méthylbenzène, 1,5-diisocyanatonaphtalène, 1,1'-(1-méthyléthylidène)bis[4-isocyanatobenzène) et 1,3- et 1,4-bis(1-isocyanato-1-méthyléthyl)benzène.

10. Composition selon la revendication 1, dans laquelle le composé oligomérique bioabsorbable qui est coiffé en extrémité avec un diisocyanate aromatique est présent en une quantité de 50 à 95 pour cent en poids de la composition;
le composé trifonctionnel qui est coiffé en extrémité avec un diisocyanate aromatique est présent en une quantité de 5 à 40 pour cent en poids de la composition; et
le diisocyanate aromatique est présent en une quantité de 1 à 10 pour cent en poids de la composition.

11. Composition selon la revendication 1, dans laquelle le composé oligomérique bioabsorbable qui est coiffé en extrémité avec un diisocyanate aromatique est présent en une quantité de 70 à 90 pour cent en poids de la composition;
le composé trifonctionnel qui est coiffé en extrémité avec un diisocyanate aromatique est présent en une quantité de 8 à 25 pour cent en poids de la composition; et
le diisocyanate aromatique est présent en une quantité de 2 à 5 pour cent en poids de la composition.

12. Composition selon la revendication 1, qui est pour faire adhérer des première et seconde surfaces de tissu, dans un procédé comprenant le rapprochement des première et seconde surfaces de tissu; et l'application aux surfaces de tissu rapprochées d'une composition de la revendication 1.

13. Composition selon la revendication 1, qui est pour faire adhérer un dispositif chirurgical à du tissu, dans un procédé comprenant: appliquer au dispositif chirurgical une composition selon la revendication 1; et mettre en contact le dispositif chirurgical avec le tissu.

14. Composition selon la revendication 1, qui est pour l'obturation d'un défaut dans le tissu dans un procédé comprenant l'identification d'un site de tissu contenant un défaut; et appliquer une composition de la revendication 1 au site du défaut.

15. Composition selon la revendication 1, qui est pour la réduction d'une fuite de fluides corporels ou d'air dans un procédé comprenant l'application à un défaut de tissu d'une composition selon la revendication 1 et la réticulation de la composition.

16. Composition comme dans la revendication 1, dans laquelle:
i) le composé oligomérique bioabsorbable présente la structure:
[A]ₙ-X
où A est un groupe bioabsorbable dérivé d'un monomère sélectionné dans le groupe consistant en acide glycolique, glycolide, acide lactique, lactide, 1,4-dioxane-2-one, 1,3-dioxane-2-one et ε-caprolactone, n est d'environ 1 à environ 6 et X est un résidu d'un initiateur multifonctionnel sélectionné dans le groupe consistant en éthylène glycol, diéthylène glycol, 1,3-propanediol, 1,4-butanediiol, 1,5-pentanediol, 1,6-hexanediol, 1,7-heptanediol, 1,8-octanediol, 1,10-décanediol, 1,12-dodécanediol, 1,2-décanediol, 1,2-dodécanediol, 1,2-hexadécanediol, néopentyl glycol, 3-méthyl-1,5-pentanediol, 2-méthyl-1,3-propanediol, 2-butyl-2-éthyl-1,3-propanediol, 2-éthyl-3-butyl- 1,3-propanediol, 2-éthyl-1,6-hexanediol, glycérol, 1,1,1-triméthylolpropane, néopentyl glycol, pentaérythritol, triéthanolamine, 1-aminopropanols, 2-aminopropanols, 2-aminobutanols, 4-aminobutanols, acide succinique, acide glutarique, acide adipique, acide subérique, acide sébacique, acide dodécanedioïque, acide 2-éthyl-2-méthylsuccinique, acide phtalique, acide isophtalique et acide téréphtalique;
(ii) le composé trifonctionnel est sélectionné dans le groupe consistant en glycérol, 1,1,1-triméthylolpropane, triéthanolamine, 1-aminopropanols, 2-aminopropanols, 2-aminobutanols, 4-aminobutanols; et
(iii) le diisocyanate aromatique est sélectionné dans le groupe consistant en
(a) polyisocyanates aromatiques contenant 6 à 20 atomes de carbone, non incluant les atomes de carbone dans les groupes NCO;
(b) polyisocyanates araliphatiques contenant 8 à 15 atomes de carbone; et
(c) polyisocyanates modifiés de ces polyisocyanates, contenant des groupes d'uréthane, carbodiimide, allophanate, urée, biuret, urethdione, urethimine, isocyanurate et/ou oxazolidone.

17. Composition telle que revendiquée dans l'une quelconque des revendications 12 à 14, dans laquelle le procédé comprend
(i) l'application au tissu d'une composition telle que définie dans la revendication 1 et
(ii) la réticulation de la composition.

18. Composition selon la revendication 15 ou la revendication 17, dans laquelle la réticulation comprend la mise en contact de la composition avec un composé sélectionné dans le groupe consistant en eau, diéthylène glycol et polyéthylène glycol.

19. Composition selon la revendication 15 ou la revendication 17, où la réticulation inclut l'utilisation d'un catalyseur.

20. Composition selon la revendication 15 ou la revendication 17, où la réticulation est effectuée à des températures de 20°C à 40°C pendant une durée de trente secondes à une heure.

21. Composition selon la revendication 15 ou la revendication 17, où la réticulation est effectuée au moins partiellement avant l'application au tissu.
